(19) Europäisches Patentamt

European Patent Office

Office européen des brevets

(11) **EP 1 924 199 B1**

(12) **FASCICULE DE BREVET EUROPEEN**

(45) Date de publication et mention
de la délivrance du brevet:
**02.03.2011 Bulletin 2011/09**

(21) Numéro de dépôt: **06794282.1**

(22) Date de dépôt: **03.08.2006**

(51) Int Cl.:
***A61B 5/103*** *(2006.01)*

(86) Numéro de dépôt international:
**PCT/FR2006/001891**

(87) Numéro de publication internationale:
**WO 2007/020341 (22.02.2007 Gazette 2007/08)**

(54) **SYSTEME IMAGEUR D'UN ORGANE HUMAIN OU ANIMAL PERMETTANT LA MESURE DE L'ELASTICITE DUDIT ORGANE**

SYSTEM ZUR DARSTELLUNG VON MENSCHLICHEN ODER TIERISCHEN ORGANEN ZUR MESSUNG DER ELASTIZITÄT DES ORGANS

HUMAN OR ANIMAL ORGAN IMAGING SYSTEM WHICH CAN BE USED TO MEASURE THE ELASTICITY OF THE ORGAN

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **12.08.2005 FR 0552503**

(43) Date de publication de la demande:
**28.05.2008 Bulletin 2008/22**

(73) Titulaire: **Echosens**
**75005 Paris (FR)**

(72) Inventeurs:
• **SANDRIN, Laurent**
**F-94240 l'Hay-les-Roses (FR)**

• **HASQUENOPH, Jean-Michel**
**F-77860 Couilly-Pont-aux-Dames (FR)**

(74) Mandataire: **Lebkiri, Alexandre**
**Cabinet Camus Lebkiri**
**10 rue de la Pépinière**
**75008 Paris (FR)**

(56) Documents cités:
**FR-A- 2 843 290        FR-A- 2 850 265
US-A- 5 115 808        US-A1- 2002 040 187
US-A1- 2002 068 870**

EP 1 924 199 B1

**Description**

L'invention concerne un transducteur ultrasonore destiné à la formation de l'image d'un organe humain ou animal permettant en outre la mesure de l'élasticité dudit organe ainsi qu'un système imageur d'un organe humain ou animal comprenant un tel transducteur.

Il est connu de mesurer l'élasticité d'un organe humain ou animal au moyen d'une vibration basse fréquence se propageant dans le corps humain ou animal vers cet organe. La vibration est engendrée par une impulsion basse fréquence effectuée à l'aide, par exemple, d'un transducteur ultrasonore. Le transducteur permet d'observer la propagation de la vibration et de former une image de l'organe dont on mesure l'élasticité par illumination ultrasonore. Un tel dispositif est par exemple décrit dans le document FR-2 843 290.

Ce document enseigne que le transducteur ultrasonore engendrant la vibration basse fréquence peut être de faible dimension afin de permettre au dispositif d'être positionné dans des espaces de dimension réduite, tels que l'espace intercostale si l'on souhaite mesure l'élasticité du foie par exemple. Cependant, afin d'obtenir une image ultrasonore correctement résolue, le transducteur devrait présenter une grande dimension car la tâche focale ultrasonore a une largeur inversement proportionnelle à cette dimension.

Le document FR-2 843 290 enseigne également que l'impulsion basse fréquence peut être générée par une barrette échographique standard que l'on met en mouvement. Cependant, la barrette échographique présentant une grande dimension, l'image ultrasonore est correctement résolue, mais la vibration basse fréquence subit des effets de diffraction et on ne peut pas introduire la barrette échographique dans un espace de dimension réduite. Le document US 2002/068870 décrit également un dispositif selon le préambule de la revendication 1.

L'invention vise à pallier ces inconvénients en proposant un transducteur dont la dimension est adaptée pour obtenir une image correctement résolue et dont au moins une partie de faible surface est mobile afin d'engendrer une vibration ultrasonore permettant en outre de mesurer l'élasticité de cet organe en évitant les effets de diffraction et en pouvant être introduite dans un espace de faible dimension.

Le transducteur ultrasonore est utilisé dans un système imageur. Dans une telle réalisation, il se pose des problèmes de calcul des retards et des amplitudes car il faut prendre en compte le déplacement de la partie mobile par rapport aux parties fixes qui entraîne un décalage dans l'émission et la réception des ultrasons. L'invention propose un système dont les moyens d'analyse permettent de prendre en compte ce déplacement.

À cet effet et selon un premier aspect, l'invention concerne un transducteur ultrasonore destiné à la formation de l'image d'un organe humain ou animal permettant en outre la mesure de l'élasticité dudit organe, ledit transducteur comprenant au moins une partie mobile (3) agencée pour induire la propagation d'une vibration basse fréquence vers l'organe lorsque la partie mobile (3) est actionnée et occasionne un choc sur le corps humain ou animal, le transducteur (2) comprenant en outre au moins une partie fixe (4).

Ainsi, l'invention permet de prendre en compte les contraintes liées à la faible dimension que doit présenter la surface engendrant la vibration basse fréquence et à la grande dimension du transducteur ultrasonore permettant d'obtenir une image ultrasonore correctement résolue.

Selon un deuxième aspect, l'invention concerne un système imageur d'un organe humain ou animal permettant en outre la mesure de l'élasticité dudit organe, comprenant un transducteur tel que décrit ci-dessus, ledit système comprenant en outre un dispositif de commande et de calcul agencé pour commander l'émission et la réception d'ultrasons et le déplacement de la partie mobile, ledit dispositif comprenant des moyens d'analyse des ultrasons émis et reçu par le transducteur et du déplacement de la partie mobile de sorte à établir l'image de l'organe et lesdits moyens d'analyse permettant en outre l'analyse de la vibration basse fréquence induite de sorte à mesurer l'élasticité dudit organe. Selon une réalisation, le système comprend un capteur de position de la partie mobile. Ainsi, les moyens d'analyse comprennent des modules d'ajustement des retards à l'émission et à la réception des signaux ultrasonores émis et reçus par la partie mobile lorsque ladite partie se déplace de sorte à faire concorder ces signaux avec les signaux émis et reçus par la partie fixe, lesdits moyens utilisant en temps réel les informations fournies par le capteur de position. On assure ainsi une concordance entre les signaux émis et reçus par la partie mobile et ceux émis et reçus par la partie fixe et le déplacement de la partie mobile est bien pris en compte pour établir l'image de l'organe humain ou animal.

D'autres aspects et avantages de l'invention apparaîtront à la lecture de la description qui suit, faite en référence aux figures annexées.

La figure 1 est une représentation schématique d'un système imageur selon l'invention.

La figure 2 est une représentation schématique du transducteur ultrasonore de la figure 1, illustrant le déplacement que peut subir la partie mobile.

La figure 3 est une représentation schématique en perspective du transducteur ultrasonore selon l'invention.

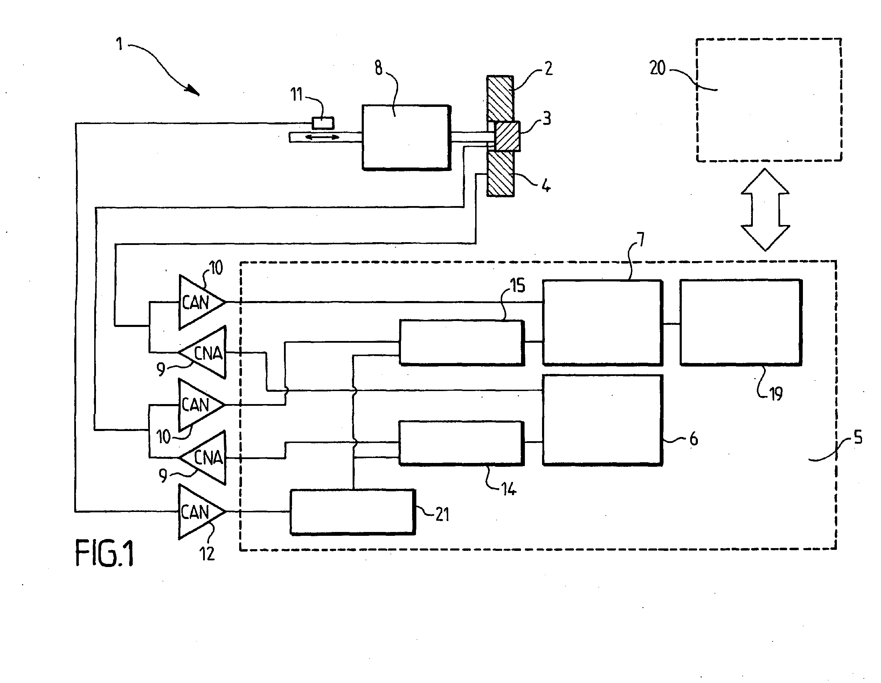

En référence à la figure 1, on décrit un système imageur d'un organe humain ou animal 1 permettant en outre la mesure de l'élasticité dudit organe. Le système 1 comprend un transducteur ultrasonore 2 de dimension générale adaptée pour obtenir une image ultrasonore correctement résolue. A cet effet, le transducteur présente par exemple la dimension d'une barrette échographique standard.

Le transducteur 2 comprend au moins une partie mobile 3 et au moins une partie fixe 4. Selon la réalisation représentée sur les figures, la partie mobile 3 se déplace en translation selon une direction sensiblement perpendiculaire à la direction dans laquelle s'étend le transducteur 2. Selon d'autres réalisations non représentées, la partie mobile 3 peut se déplacer en translation selon la direction dans laquelle s'étend le transducteur ou se déplacer en rotation de sorte à engendrer la vibration basse fréquence.

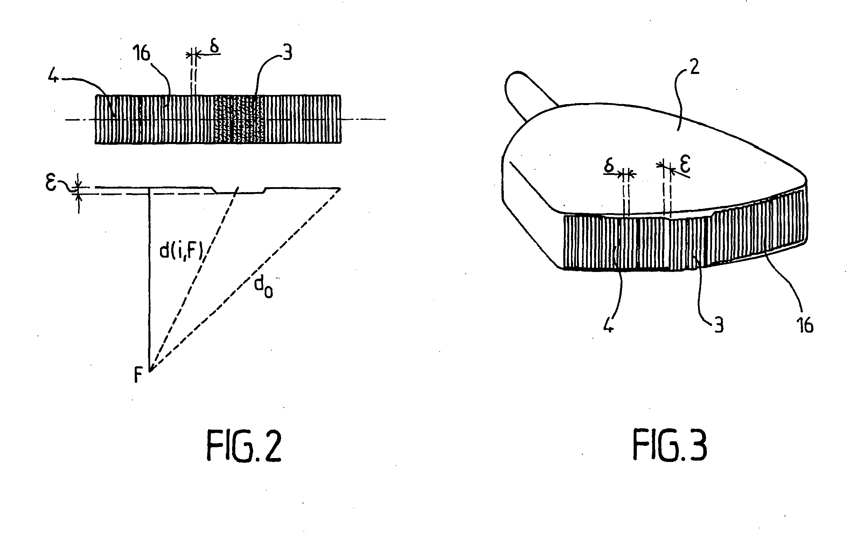

La dimension de la partie mobile 3 est réduite par rapport à la dimension du transducteur 2. En particulier, la partie mobile 3 présente une surface adaptée pour limiter les effets de diffraction lors de la propagation de la vibration basse fréquence et pour que la partie mobile puisse être introduite dans l'espace intercostal du corps humain ou animal. La partie mobile est par exemple disposée sensiblement au centre du transducteur 2 entre deux parties fixes 4, comme représenté sur les figures 1 et 3.

Selon une réalisation non représentée, le transducteur 2 peut comprendre une pluralité de parties mobiles 3 réparties en alternance avec des parties fixes 4 le long du transducteur 2. Selon une autre réalisation, le transducteur 2 peut comprendre des parties mobiles 3 disposées les unes à côté des autres.

Le système 1 comprend des moyens de mise en mouvement 8 de la partie mobile 3. De la sorte, la partie mobile est agencée pour induire la propagation d'une vibration basse fréquence vers l'organe lorsque la partie mobile 3 est actionnée en translation et occasionne un choc sur le corps humain ou animal. Les moyens de mise en mouvement 8 sont commandés par un dispositif de commande et de calcul 5 qui commande en outre l'émission d'ultrasons par la partie fixe 4 et par la partie mobile 3. Dans le cas où plusieurs parties mobiles 3 sont prévues, le dispositif de commande et de calcul 5 peut commander les moyens de mise en mouvement 8 de sorte que les parties mobiles 3 se déplacent en opposition de phase.

Le dispositif de commande et de calcul 5 comprend un module de génération des signaux d'émission 6 et un module de formation de voies 7. Le module de génération 6 fournit un signal d'émission ultrasonore à la partie mobile 3 et à la partie fixe 4 au moyen d'une loi d'émission choisie pour permettre de former une image correctement résolue de l'organe observé. Le module de génération 6 est relié à la partie fixe 4 et à la partie mobile 3 par l'intermédiaire de convertisseurs numérique analogique 9, comme représenté sur la figure 1. De même, la partie fixe 4 et la partie mobile 3 sont reliées au module de formation de voies 7 par l'intermédiaire de convertisseurs analogique numérique 10. Le module de formation de voies 7 peut ensuite être relié à un module de calcul de l'élasticité 19. Le module de commande et de calcul 5 est relié à un dispositif d'affichage 20 permettant de visualiser l'image de l'organe et les résultats de la mesure de l'élasticité de l'organe ou à un système d'exploitation, une interface avec un utilisateur par exemple.

Un capteur de position 11 de la partie mobile 3 est associé à cette partie mobile 3. Le capteur de position 11 permet de relever la position de la partie mobile 3, lorsque celle-ci se déplace et n'est plus alignée avec la partie fixe 4. Le déplacement de la partie mobile 3 peut se faire sur une distance E, comme représenté sur la figure 2. Le capteur de positon 11 est, par exemple, un capteur à effet Hall relié au module de génération 6 et au module de formation de voies 7 par l'intermédiaire d'un convertisseur analogique numérique 12 apte à numériser le signal fourni par ce capteur. D'autre part, le dispositif de commande et de calcul 5 comprend des moyens de génération et de traitement des ultrasons émis et reçu par le transducteur de sorte à établir l'image de l'organe et lesdits moyens de génération et de traitement permettant en outre l'analyse de la vibration basse fréquence induite de sorte à mesurer l'élasticité dudit organe. Ces moyens de génération et de traitement 6 et 7 sont associés à des modules d'ajustement en temps réel des retards 14 et 15 à l'émission et à la réception des signaux ultrasonores émis et reçus par la partie mobile lorsque ladite partie se déplace de sorte à faire concorder ces signaux avec les signaux émis et reçus par la partie fixe. Ils comprennent en outre des modules d'ajustement en temps réel des gains 14 et 15 à l'émission des signaux ultrasonores émis par la partie mobile lorsque ladite partie se déplace de sorte à faire concorder ces gains avec les gains de signaux ultrasonores émis par la partie fixe. Ces modules d'ajustement 14 et 15 sont disposés entre un module de calcul des correction 21 et le module de génération 6 et le module de formation de voies 7, comme représenté sur la figure 1, et leur fonctionnement est décrit ci-dessous.

Le capteur de position 11 délivre un signal représentatif du déplacement ε de la partie mobile 3. Ce signal est numérisé au travers du convertisseur analogique numérique 12 puis communiqué au module de calcul des corrections 21 qui utilise en temps réel les informations fournies par le capteur de position 11. Les corrections sont fournies aux modules d'ajustement 14 et 15. Le transducteur ultrasonore 2 est un transducteur classique comprenant une pluralité d'éléments 16 pouvant émettre et recevoir des ultrasons. On note $\delta$ le pas entre chaque élément 16, c'est-à-dire la distance séparant deux éléments consécutifs. D'autre part, on note F le point focal situé sur la ligne formée par le transducteur 2. On note $d(i, F)$ la distance entre le point focal F et un élément i du transducteur 2 et $d_o$ la plus grande distance entre un élément 16 de l'ouverture utilisée et le point focal F. Vs est la vitesse de ultrasons se propageant dans le corps.

Selon les notations ci-dessus, les retards à l'émission $R_e$ et les retards à la réception $R_r$ pour un élément i immobile et pour la construction d'une ligne numérotée c se calculent de la façon suivante :

$$\begin{cases} R_e(c,i) = \dfrac{d_0 - d(i,F)}{V_s} \\ R_r(c,i) = \dfrac{d(i,F)}{V_s} \end{cases}$$

Pour un élément i décalé d'une distance ε par rapport à la position dans laquelle il est aligné avec les éléments 16 de la partie fixe, les retards corrigés se calculent de la façon suivante :

$$\begin{cases} R_e(c,i) = \dfrac{d_0 - \sqrt{[F-\varepsilon]^2 + [(i-c)\delta]^2}}{V_s} \\ R_r(c,i) = \dfrac{\sqrt{[F-\varepsilon]^2 + [(i-c)\delta]^2}}{V_s} \end{cases}$$

Les corrections par rapport aux retards appliqués lorsque le déplacement ε est nul, s'expriment de la façon suivante lorsque le déplacement ε reste négligeable par rapport à la distance focale :

$$\begin{cases} \Delta R_e(c,i) = \dfrac{\varepsilon}{V_s}\left(1 + \dfrac{[(i-c)\delta]^2}{F^2}\right)^{-\frac{1}{2}} \\ \Delta R_r(c,i) = -\Delta R_e(c,i) \end{cases}$$

Le module de calcul des correction 21 permet d'effectuer ces calculs et d'en fournir les résultats aux modules d'ajustement à l'émission et à la réception14 et 15 comme représenté sur la figure 1. Dans le module de formation de voies 7, un sommateur permet d'additionner les signaux reçus par la partie fixe 4 et ceux reçus par la partie mobile 3 afin de former l'image de l'organe.

Les données fournies par le capteur de position 11 sont utilisées en temps réel de sorte à imager l'organe à tout instant sans délai important de traitement.

Les modules d'ajustement 14 et 15 permettent également l'adaptation du gain à l'émission des signaux ultrasonores émis par la partie mobile 3 en fonction de sa position, comme représenté sur la figure 1. En effet, la partie mobile 3 n'émettant pas les signaux depuis le même endroit que la partie fixe 4, il faut adapter l'amplitude des signaux émis par la partie mobile 3 de sorte à ce qu'elle soit égale à celle des signaux émis par la partie fixe 4.

La mesure de l'élasticité de l'organe au moyen du système 1 est connue en elle-même et peut, par exemple, s'inspirer de la solution proposée par le document FR-2 843 290.

**Revendications**

1. Transducteur ultrasonore destiné à la formation de l'image d'un organe humain ou animal permettant en outre la mesure de l'élasticité dudit organe, ledit transducteur étant **caractérisé en ce que** qu'il comprend au moins une partie mobile (3) agencée pour induire la propagation d'une vibration basse fréquence vers l'organe lorsque la partie mobile (3) est actionnée et occasionne un choc sur le corps humain ou animal **caractérisé en ce que** le transducteur (2) comprenne en outre au moins une partie fixe (4).

2. Transducteur selon la revendication 1, **caractérisé en ce qu'**il comprend un moyen de mise en mouvement (8) de la partie mobile (3).

3. Transducteur selon la revendication 1 ou 2, **caractérisé en ce que** la partie mobile (3) est déplaçable en translation.

4. Transducteur selon la revendication 1 ou 2, **caractérisé en ce que** la partie mobile (3) est déplaçable en rotation.

5. Transducteur selon l'une quelconque des revendications 1 à 4, **caractérisé en ce que** la partie mobile (3) présente une surface adaptée pour limiter les effets de diffraction lors de la propagation de la vibration basse fréquence et pour que la partie mobile (3) puisse être introduite dans l'espace intercostal du corps humain ou animal.

6. Transducteur selon l'une quelconque des revendications 1 à 5, **caractérisé en ce qu'**il comprend une pluralité de parties mobiles (3) et une pluralité de parties fixes (4).

7. Système imageur d'un organe humain ou animal permettant en outre la mesure de l'élasticité dudit organe, **caractérisé en ce qu'**il comprend un transducteur (2) selon l'une quelconque des revendications 1 à 6, ledit système comprenant en outre un dispositif de commande et de calcul (5) agencé pour commander l'émission et la réception d'ultrasons et le déplacement de la partie mobile (3), ledit dispositif comprenant des moyens de génération et de traitement des ultrasons émis et reçu par le transducteur (2) et du déplacement de la partie mobile de sorte à établir l'image de l'organe.

8. Système selon la revendication 7, **caractérisé en ce qu'**il comprend un capteur de position (11) de la partie mobile (3).

9. Système selon la revendication 8, **caractérisé en ce que** le capteur de position (11) est un capteur à effet Hall, le dispositif de commande et de calcul (5) comprenant un convertisseur analogique numérique (12) apte à numériser le signal fourni par ledit capteur.

10. Système selon la revendication 8 ou 9, **caractérisé en ce que** les moyens de génération et de traitement comprennent des modules d'ajustement des retards (14, 15) à l'émission et à la réception des signaux ultrasonores émis et reçus par la partie mobile (3) lorsque ladite partie se déplace de sorte à faire concorder ces signaux avec les signaux émis et reçus par la partie fixe (4), lesdits modules utilisant en temps réel les informations fournies par le capteur de position (11) par l'intermédiaire d'un module de calcul des corrections (21).

11. Système selon l'une quelconque des revendications 8 à 10, **caractérisé en ce que** les moyens de génération et de traitement comprennent des modules d'ajustement des gains (14, 15) à l'émission des signaux ultrasonores émis par la partie mobile (3) lorsque ladite partie se déplace de sorte à faire concorder ces gains avec les gains de signaux ultrasonores émis par la partie fixe (4), lesdits modules utilisant en temps réel à partir des informations fournies par le capteur de position (11) par l'intermédiaire d'un module de calcul des corrections (21).

**Claims**

1. An ultrasonic transducer intended for forming an image of a human or animal organ, also allowing the elasticity of said organ to be measured, said transducer being **characterized in that** the transducer comprises at least one mobile part (3) arranged to induce the propagation of a low frequency vibration in the direction of the organ when the mobile part (3) is activated and produces an impact against the human or animal body, **characterized in that** the transducer (2) also comprises at least one fixed part (4).

2. The transducer according to claim 1, **characterized in that** the transducer comprises means for activating (8) the mobile part (3).

3. The transducer according to claim 1 or 2, **characterized in that** the mobile part (3) is displaceable in translation.

4. The transducer according to claim 1 or 2, **characterized in that** the mobile part (3) is displaceable in rotation.

5. The transducer according to any one of claims 1 to 4, **characterized in that** the mobile part (3) presents a surface adapted to limit diffraction effects during propagation of the low frequency vibration and so that the mobile part (3) may be introduced in the intercostal space of the human or animal body.

6. The transducer according to any one of claims 1 to 5, **characterized in that** the transducer comprises a plurality of mobile parts (3) and a plurality of fixed parts (4).

7. An imaging system for a human or animal organ also allowing the elasticity of said organ to be measured, **characterized in that** the system comprises a transducer (2) according to any one of claims 1 to 6, said system also comprising a control and calculation device (5) arranged to control the ultrasound transmission and reception and the displacement of the mobile part (3), said device comprising means for generating and processing the ultrasound transmitted and received by the transducer (2) and the displacement of the mobile part so as to create the organ image.

8. The system according to claim 7, **characterized in that** the system comprises a position sensor (11) of the mobile part (3).

9. The system according to claim 8, **characterized in that** the position sensor (11) is a Hall effect sensor, the control and calculation device (5) comprising an analog-to-digital converter (12) able to digitize the signal provided by said sensor.

10. The system according to claim 8 or 9, **characterized in that** the generation and processing means comprise modules (14, 15) for adjusting delays in the transmission and reception of ultrasound signals transmitted and received by the mobile part (3) when said part is displaced so as to make such signals coincide with the signals transmitted and received by the fixed part (4), said modules utilizing in real time data provided by the position sensor (11) through a correction calculating module (21).

11. The system according to any one of claims 8 to 10, **characterized in that** the generation and processing means comprise modules (14, 15) for adjusting gains in the transmission of ultrasound signals transmitted by the mobile part (3) when said part is displaced so as to make these gains coincide with the gains from ultrasound signals transmitted by the fixed part (4), said modules utilizing in real time data provided by the position sensor (11) through a correction calculating module (21).

**Patentansprüche**

1. Ultraschallwandler, der zur Bildung eines Bildes eines menschlichen oder tierischen Organs bestimmt ist und der ferner die Messung der Elastizität des Organs ermöglicht, wobei der Wandler **dadurch gekennzeichnet ist, dass** er mindestens einen beweglichen Abschnitt (3) umfasst, der gestaltet ist, um die Ausbreitung einer niederfrequenten Schwingung zum Organ zu bewirken, wenn der bewegliche Abschnitt (3) betätigt wird und einen Stoß auf dem menschlichen oder tierischen Körper verursacht; **dadurch gekennzeichnet, dass** der Wandler (2) ferner mindestens einen festen Abschnitt (4) umfasst.

2. Wandler nach Anspruch 1, **dadurch gekennzeichnet, dass** er ein Mittel zur Inbewegungsetzung (8) des beweglichen Abschnitts (3) umfasst.

3. Wandler nach Anspruch 1 oder 2, **dadurch gekennzeichnet, dass** der bewegliche Abschnitt (3) translatorisch verschiebbar ist.

4. Wandler nach Anspruch 1 oder 2, **dadurch gekennzeichnet, dass** der bewegliche Abschnitt (3) drehbar verschiebbar ist.

5. Wandler nach einem der Ansprüche 1 bis 4, **dadurch gekennzeichnet, dass** der bewegliche Abschnitt (3) eine Fläche aufweist, die angepasst ist, um die Diffraktionseffekte bei der Ausbreitung der niederfrequenten Schwingung zu begrenzen und damit der bewegliche Abschnitt (3) in den Interkostalraum des menschlichen oder tierischen Körpers eingeführt werden kann.

6.  Wandler nach einem der Ansprüche 1 bis 5, **dadurch gekennzeichnet, dass** er mehrere bewegliche Abschnitte (3) und mehrere feste Abschnitte (4) umfasst.

7.  System zur Aufnahme eines Bildes eines menschlichen oder tierischen Organs, das ferner die Messung der Elastizität des Organs ermöglicht, **dadurch gekennzeichnet, dass** es einen Wandler (2) nach einem der Ansprüche 1 bis 6 umfasst, wobei das System ferner eine Steuerungs- und Berechnungsvorrichtung (5) umfasst, die gestaltet ist, um die Sendung und den Empfang von Ultraschall und die Verschiebung des beweglichen Abschnitts (3) zu steuern, wobei die Vorrichtung Mittel zur Erzeugung und Verarbeitung von durch den Wandler (2) ausgesendetem und empfangenem Ultraschall und zur Verschiebung des beweglichen Abschnitts umfasst, derart, dass ein Bild des Organs erstellt wird.

8.  System nach Anspruch 7, **dadurch gekennzeichnet, dass** es einen Sensor (11) für die Position des beweglichen Abschnitts (3) umfasst.

9.  System nach Anspruch 8, **dadurch gekennzeichnet, dass** der Positionssensor (11) ein Hall-Effekt-Sensor ist, wobei die Steuerungs- und Berechnungsvorrichtung (5) einen Analog-Digital-Wandler (12) umfasst, der in der Lage ist, das durch den Sensor gelieferte Signal zu digitalisieren.

10. System nach Anspruch 8 oder 9, **dadurch gekennzeichnet, dass** die Erzeugungs- und Verarbeitungsmittel Module zur Berichtigung der Verzögerungen (14, 15) bei der Aussendung und beim Empfang der durch den beweglichen Abschnitt (3) ausgesendeten und empfangenen Ultraschallsignale umfassen, wenn der Abschnitt sich derart verschiebt, dass diese Signale mit den durch den festen Abschnitt (4) ausgesendeten und empfangenen Signalen in Übereinstimmung gebracht werden, wobei die Module die durch den Positionssensor (11) gelieferten Informationen über ein Modul zur Berechnung der Korrekturen (21) in Echtzeit verwenden.

11. System nach einem der Ansprüche 8 bis 10, **dadurch gekennzeichnet, dass** die Erzeugungs- und Verarbeitungsmittel Module zur Berichtigung der Verstärkungen (14, 15) bei der Aussendung der durch den beweglichen Abschnitt (3) ausgesendeten Ultraschallsignale umfassen, wenn der Abschnitt sich derart bewegt, dass diese Verstärkungen mit den Verstärkungen von durch den festen Abschnitt (4) ausgesendeten Ultraschallsignalen in Übereinstimmung gebracht werden, wobei die Module die durch den Positionssensor (11) gelieferten Informationen über ein Modul zur Berechnung der Korrekturen (21) in Echtzeit verwenden.

FIG.1

FIG.2

FIG.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2843290

- US 2002068870 A